# EUROPÄISCHE PATENTSCHRIFT

(11) **EP 3 100 762 B1**
(45) Veröffentlichungstag und Bekanntmachung des Hinweises auf die Patenterteilung: **01.04.2020**
(21) Anmeldenummer: 16170560.3
(22) Anmeldetag: 20.05.2016
(51) Int. Cl.: A61N 1/04, A61N 1/08, A61N 1/18

(54) **DIAGNOSTISCHES MEDIZINPRODUKT MIT EINER HAFTVERMITTELNDEN OBERFLÄCHENSTRUKTUR**
DIAGNOSTIC MEDICAL PRODUCT HAVING AN ADHESION-ENHANCING SURFACE STRUCTURE
PRODUIT MEDICAL DIAGNOSTIC COMPRENANT UNE STRUCTURE DE SURFACE ADHESIVE

(30) Priorität: 02.06.2015 DE 102015108670; 02.06.2015 DE 102015108672; 02.06.2015 DE 102015108671
(43) Veröffentlichungstag der Anmeldung: 07.12.2016
(73) Patentinhaber: BIOTRONIK SE & Co. KG, 12359 Berlin (DE)
(72) Erfinder: Kolberg, Gernot, 12161 Berlin (DE); Friedrich, Michael, 14532 Kleinmachnow (DE)
(74) Vertreter: Galander, Marcus

(56) Entgegenhaltungen:
- WO-A1-2014/178943
- US-A1- 2006 047 215
- US-A1- 2006 204 738
- US-A1- 2011 130 804
- US-A1- 2014 276 407

## Beschreibung

Die vorliegende Erfindung betrifft ein therapeutisches oder diagnostisches Medizinprodukt, das mindestens auf Teilen seiner nach außen gewandten Oberfläche eine haftvermittelnde Struktur aufweist.

Die temporäre bis dauerhafte Fixierung von therapeutischen oder diagnostischen Medizinprodukten an Mensch oder Tier ist von großer Bedeutung. Eine solche Fixierung soll insbesondere einer Dislokation der Medizinprodukte im oder am Körper vorbeugen, in deren Folge die gewünschte diagnostische oder therapeutische Funktion nicht mehr gewährleistet werden kann oder gar zusätzliche medizinische Komplikationen auftreten. Der Fixiermechanismus selbst sollte möglichst geringe Auswirkung auf den Organismus haben. Bei rein mechanischer Fixierung durch Einnähen, Verankerungsstrukturen oder Klemmelementen kann das betroffene Gewebe nachhaltig und möglicherweise irreparabel verletzt werden. Haftvermittelnde Klebstoffe können zu Unverträglichkeitsreaktionen führen und damit fixierte Medizinprodukte lassen sich in der Regel nicht mehr verletzungsfrei von dem anhaftenden Gewebe trennen.

Ein anderer Grund, Komponenten im Gefäßsystem oder im oder auf dem Herzen zu fixieren, ist der, die Komponenten an einer sicheren Position zu halten. Andernfalls würden die Komponenten vom Blutstrom mitgespült werden oder durch die Schwerkraft an Orte gelangen, wo sie dem Patienten gefährlich werden können. So können sie Gefäße verstopfen und Embolien, Herzinfarkt oder Gehirnschlag verursachen.

Die PCT-Anmeldung WO2014178943A1 beschreibt eine Vorrichtung mit einer Vielzahl von ersten Reservoirs und einer Vielzahl von zweiten Reservoirs die mit einem Substrat verbunden sind.

Es besteht daher ein Bedürfnis nach alternativen Fixiermechanismen für therapeutische oder diagnostische Medizinprodukte, die einerseits eine zuverlässige und störungsarme Fixierung gewährleisten, aber anderseits wieder rückstandfrei lösbar sind.

Die geschilderten Probleme des Standes der Technik lassen sich mit Hilfe des erfindungsgemäßen implantierbaren Sensors nach Anspruch 1 lösen oder zumindest mindern. Das Medizinprodukt weist dazu eine haftvermittelnde Oberflächenstruktur - vorzugsweise Geckostruktur - auf, die Teil einer elastischen Membran ist, die eine mit einem plastisch verformbaren Material befüllte Kavität abdeckt.

Die Erfindung nutzt somit eine alternative Möglichkeit der Verbindung unterschiedlicher Oberflächen über das Phänomen der Trockenadhäsivität. Unter Trockenadhäsivität wird vorliegend die Ausbildung von adhäsiven Kräften zwischen Oberflächen ohne haftvermittelnde Stoffe, wie Klebstoffe, verstanden. Solche Haftsysteme sind beispielsweise auch aus der Natur, z.B. bei Gecko- oder Insekten-Beinen, bekannt. Man nimmt an, dass bei solchen Systemen die Haftkräfte auf van-der-Waals-Kräften basieren. Die hafterzeugende Oberfläche weist dazu eine haftvermittelnde Oberflächenstruktur auf, beispielsweise eine Vielzahl von borsten- oder haarförmigen Elemente, die zu einer sehr starken Vergrößerung der zur Verfügung stehenden Kontaktfläche führen. Mit der Vergrößerung der Kontaktfläche nimmt in der Folge die Stärke der bei der Kontaktierung ausgebildeten Adhäsionskräfte zu. Der Einsatz derartiger haftvermittelnder Oberflächenstrukturen zur Anbindung an Gewebe wird beispielsweise von Alborz Mahdavi et al., ,A biodegradable and biocompatible gecko-inspired tissue adhesive', PNAS (2008), Vol. 105, No. 7, 2307-2312, vorgeschlagen.

Bei der Trockenadhäsivität hängt demnach die Stärke der Adhäsion zwischen zwei Oberflächen mit der für die Adhäsion verfügbaren Fläche zusammen. Zwei planare Oberflächen haften deutlich besser aneinander, als eine planare und eine raue oder nicht planare Oberfläche. Allgemein gilt, je grösser die zur Adhäsion verfügbare Fläche ist, desto besser haften zwei Oberflächen aneinander. Allerdings ist diese Fläche bisher nicht veränderbar, so dass die Adhäsionskraft nicht veränderbar ist. Dadurch, dass die erfindungsgemäße haftvermittelnde Oberflächenstruktur Teil einer Membran ist, die locker die mit dem plastisch verformbaren Material befüllte Kavität bedeckt, kann die zur Adhäsion verfügbare Fläche wesentlich erhöht werden. Mit anderen Worten, die Membran und das plastisch verformbare Material bilden eine Art Gelkissen, so dass eine Oberflächenkontur der Membran sich der Oberflächenkontur des Gewebes anpassen kann.

Die haftvermittelnde Oberflächenstruktur kann beispielsweise zwischen 10 und 1.000.000 Stäbchen pro Quadratmillimeter aufweisen. Das Verhältnis von Durchmesser und Länge der Stäbchen kann zwischen 1:2 und 1:2000 liegen. Der Querschnitt des Stäbchens kann querprofiliert sein, zum Beispiel ganz oder teilweise rund, dreieckig, rechteckig, quadratisch oder innen hohl. Er kann ein T-Profil haben oder den Umrissen einer Mondsichel entsprechen. Dadurch kann eine Vorzugsbiegerichtung des Stäbchens vorgegeben werden. Alternativ oder in Kombination können die Stäbchen vorgebogen oder schräg angebracht sein. Eine einheitliche Biegerichtung der Stäbchen kann verhindern, dass die Stäbchen sich untereinander verheddern. Die Stäbchen können ferner ein Längsprofil haben. So können sie an der Wurzel, wo sie an der zu fixierenden Komponente anliegen verdickt sein und sich zum Ende hin verjüngen.

Die haftvermittelnde Oberflächenstruktur kann aus sich verästelnden Stäbchen bestehen. Das Ende des letzten Zweiges kann wieder verdickt sein. Die größte Ausdehnung der Verdickung entspricht maximal dem 100-fachen des Stäbchendurchmessers, auf dem die Verdickung sitzt. Das Ende des letzten Zweiges kann auch planar oder abgerundet sein oder spitz auslaufen. Am Ende des letzten Zweiges kann sich eine lappenartige Struktur, ähnlich einer Kelle, befinden, die einseitig angebunden ist. Die lappenartige Struktur ist vorzugsweise einseitig an die Stäbchen angebunden in der Art, dass der Winkel der Stäbchen fortgesetzt wird. So wird bei einer Querkraft der Komponente in die ablösende Richtung (zum Beispiel entgegen dem Uhrzeigersinn) die lappenartige Struktur vom Gewebe geschält, was das Ablösen erheblich erleichtert, während bei Querkraft in die andere Richtung nur eine Scherkraft verursacht wird, die die Fixierung nicht nur nicht ablöst, sondern unterstützt.

Durch Gestaltung der Biegerichtung der Stäbchen auf der Fläche können die Fixations- und Ablösekräfte eingestellt werden. Die Strukturen fixieren besonders gut, wenn möglichst viele Stäbchen gleichzeitig die Zugkräfte aufnehmen. Soll die Fixierung gelöst werden, müssen die Stäbchen möglichst einzeln belastet werden, um auch mit geringen Kräften eine Ablösung zu ermöglichen. Durch die Vorzugsbiegerichtung der Stäbchen kann eine seitlich auf die Komponente wirkende Kraft je nach Richtung in eine Zugkraft oder in eine Druckkraft umgesetzt werden. Eine Kraft entgegen der Stäbchenausrichtung führt zu einer das Stäbchen stauchenden Kraft, die ein Umbiegen des Stäbchens verursacht, in dessen Folge an der fixierenden Oberfläche ein Abrollen stattfindet, das die fixierende Fläche abschält. Dieser Effekt kann auch über mehrere Stäbchenebenen ausgenutzt werden. So kann zum Beispiel nur das untere Ende der Stäbchen mit Vorzugsrichtung ausgestattet sein. Die darauf folgenden zum Beispiel verästelten Strukturen werden abgeschält. Ein gleichwertiger Effekt wird erzielt, wenn die Stäbchen nicht eine Vorzugsbiegerichtung haben, sondern bereits schräg angebracht oder vorgekrümmt sind.

Eine spezielle Ausführung der vorgebogenen oder mit Vorzugsbiegerichtung versehenen Stäbchen ist die, bei der die Stäbchen um einen Drehpunkt, vorzugsweise den Punkt der elektrisch aktiven beziehungsweise sensiblen Fläche in eine Richtung, vorzugsweise entgegen dem Uhrzeigersinn vorgebogen sind. Ein Drehen an der Komponente entgegen dem Uhrzeigersinn rollt jedes einzelne Stäbchenende um die Fixierstelle und schält es ab. So kann die Fixierung durch Andrücken der Komponente oder durch Drehen im Uhrzeigersinn erfolgen. Ein Ablösen erfolgt durch Drehung entgegen dem Uhrzeigersinn.

Neben der genannten tangentialen Ausrichtung der Stäbchen sind weitere Strukturierungen vorstellbar, zum Beispiel eine Fläche, deren Stäbchen in eine Richtung zeigen, ist durch eine Kraft in diese Richtung ablösbar und in die andere Richtung stabil.

Soll die Komponente mit einer orthogonal wirkenden Kraft abgelöst werden, ist es sinnvoll, dass die Stäbchen zum Membranmittelpunkt zeigen. Bei senkrechtem Abheben der Komponente lösen sich die Stäbchen von außen nach innen ab. Dieser Vorgang kann alternativ durch einen Stößel, der von innen auf die Membran drückt, oder mit Fluiddruck ausgelöst werden.

Die haftvermittelnde Oberflächenstruktur kann grundsätzlich aus jedem Material gefertigt werden, das sich mit den weiteren Bestandteilen der Membran verbinden lässt und das ausreichend verträglich ist für einen intrakorporalen Einsatz. Die haftvermittelnden Oberflächenstrukturen bestehen bevorzugt aus einem polymeren Werkstoff, insbesondere einem Silikon. Weitere mögliche Werkstoffe für die Strukturen umfassen Kohlenstoffmaterialien, insbesondere in Form von Fasern und Nanotubes, Polypropylen, Polytetrafluorethylen (PTFE), Ethylen-Tetrafluorethylen (ETFE), Polycarbonat, Polystyrol, Polylactide, wie zum Beispiel PDLLA, künstliche Spinnenseide, Polyurethane und Copolymere davon, Polyimid, Polyamid, Polyetheretherketon (PEEK), Polysulfon, Polyethylen, Polyoxymethylen (POM), Polyetherblockamid, Chitin, Kollagen, Zellulose, Keratin, Metalle, Glas und Keramik. Die haftungsvermittelnden Oberflächenstrukturen können insbesondere aus einem elektrisch leitfähig Material bestehen, um neben dem mechanisch stabilen Kontakt auch einen elektrischen Kontakt zu ermöglichen. Die Struktur kann mit einer geeigneten Substanz beschichtet werden, wie zum Beispiel Poly(dopaminmethacrylat-co-2-methoxyethylacrylat) (p(DMA-co-MEA)), um die Haftfestigkeit in flüssigen Medien zu verbessern, oder mit Steroiden, um Entzündungsprozesse zu unterdrücken. Weiterhin können Substanzen Einsatz finden, die das Einwachsverhalten fördern.

Die Membran kann mehrlagig aufgebaut sein, wobei die äußerste Lage die haftvermittelnde Oberflächenstruktur aufweist. Die eine oder mehreren inneren Lagen der Membran sind dann vorzugsweise aus einem polymeren Werkstoff, insbesondere einem Polyethylen, geformt. Die ein oder mehreren inneren Lagen der Membran lassen sich auf diese Weise unabhängig von der haftvermittelnden Oberflächenstruktur bezüglich der für die jeweilige Applikation gewünschten Eigenschaften optimieren. Die Membran kann aber gegebenenfalls auch aus demselben Material, aus dem die haftvermittelnde Oberflächenstruktur geformt ist, bestehen. In diesem Fall kann ein mehrlagiger Membranaufbau entfallen.

Eine haftvermittelnde Oberflächenstruktur kann mit unterschiedlichen Verfahren hergestellt werden. Beispielsweise können durch lithographische Verfahren, wie z. B. Elektronenstrahllithographie und Laserlithographie, oder durch Ätzverfahren negative Formen erzeugt werden. In einem anschließenden Gussverfahren wird dann ausgehend von der negativen Form die positive Oberfläche mit haarähnlichen Fortsätzen erzeugt (siehe beispielsweise A.K. Geim et al., Nature Mater. 2, 461-463 (2003) und H. Lee, B.P. Lee and P.B. Messersmith, Nature 448, 338-341 (2007)).

Vorzugsweise ist das Medizinprodukt ein implantierbarer Sensor, eine Herzelektrode, eine Herzklappe, eine Medikamentenpumpe, ein Katheter (zum Beispiel ein elektrophysiologischer Katheter, Ultraschallkatheter, Ballonkatheter, Ablationskatheter, Blasenkatheter oder RSD-Katheter), ein Stent, eine Magensonde, ein Gefäßreparaturhilfsmittel (sogenannte patches), eine externe Hautelektrode (zum Beispiel Elektroden für EKG oder Muskelstimulation), ein Heftpflaster, eine Zahnspange oder eine Prothese.

Weitere bevorzugte Ausgestaltungen der Erfindung ergeben sich aus den Unteransprüchen und der nachfolgenden Beschreibung.

Die Erfindung wird nachfolgend anhand eines Ausführungsbeispiels und dazugehöriger Zeichnungen erläutert. Es zeigen:
- Fig. 1: eine schematische Darstellung eines erfindungsgemäßen Medizinprodukts in Form eines implantierbaren Sensors;
- Fig. 2: eine Schnittansicht durch einen Teilbereich des Sensors aus Figur 1, der eine Membran mit einer haftvermittelnden Oberflächenstruktur aufweist; und
- Fig. 3: eine schematische Schnittansicht durch eine weitere Ausführungsform der Membran mit haftvermittelnder Oberflächenstruktur.

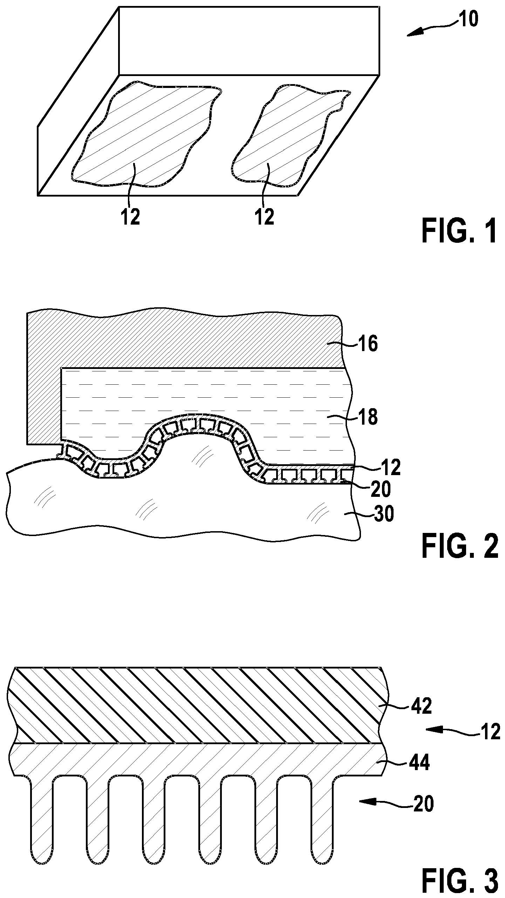

Figur 1 stellt stark schematisiert einen implantierbaren Sensor 10 dar, der mit den erfindungsgemäßen Mitteln zur intrakorporalen Fixierung ausgestattet ist. Der Sensor 10 kann beispielsweise derart ausgelegt sein, dass er über Elektroden elektrophysiologische Prozesse in einem anliegenden Gewebe detektieren kann und mit - hier nicht näher dargestellter - kabelfreier Übermittlungstechnik Daten an ein externes Lesegerät übermittelt.

Die erfindungsgemäßen Fixiermittel sind hier beispielhaft in zwei Bereichen einer Längsseite des Sensors 10 angeordnet und umfassen jeweils eine Membran 12 mit einer im Folgenden noch näher erläuterten haftvermittelnden Oberflächenstruktur. Die der schematischen Darstellung zu entnehmende unregelmäßige Randkontur der elastischen Membran 12 soll verdeutlichen, dass die Membran 12 nicht straff gespannt an dem Sensor 10 anliegt, sondern in ihrer Oberflächenkontur verformbar ist.

Der Figur 2 ist ein Teilausschnitt durch den Sensor 10 der Figur 1 zu entnehmen. Ein das erfindungsgemäße Fixiermittel tragender Gehäuseteil 16 des Sensors 10 weist dabei eine Kavität 18 auf. Diese wird von der Membran 12 hermetisch abgedeckt, wobei jedoch die Membran 12 nicht fest über die Ränder der Kavität 18 verspannt wird, sondern noch ein gewisses Spiel aufweist. Das Innere der Kavität 18 wird mit einem plastisch verformbaren Gel gefüllt. Membran 12 und die befüllte Kavität 18 bilden somit ein Gelkissen. An der Außenseite der Membran 12 befinden sich die haftvermittelnden Oberflächenstrukturen 20, die eine Anbindung an das Gewebe 30 ermöglichen sollen. Die haftvermittelnden Oberflächenstrukturen 20 bedecken vorzugweise die gesamte äußere Oberfläche der Membran 12.

Die haftvermittelnden Oberflächenstrukturen 20 liegen hier rein exemplarisch in Form von Stäbchen mit einem tellerförmig erweiterten Kopf vor. Die konkrete Ausgestaltung der haftvermittelnden Oberflächenstruktur 20 kann jedoch variieren, zum Beispiel in Abhängigkeit von der geplanten Applikation. Insbesondere wird eine der Natur nachempfundene Gecko-Struktur an der Außenseite der Membran 12 bereitgestellt. Die Herstellung der haftvermittelnden Oberflächenstruktur 20 kann, wie weiter oben bereits ausgeführt, in an sich bekannter Weise durch Erstellung passender Negativformen für ein Kunststoff-Gussverfahren erfolgen.

Wie aus der schematischen Darstellung der Figur 2 ersichtlich, passt sich die Membran 12 beim Andrücken des Sensors 10 an die Oberflächenkontur des Gewebes 30 an. Hierdurch wird die für die Adhäsion notwendige Kontaktfläche zwischen den beiden Oberflächen wesentlich erhöht.

Figur 3 ist eine weitere Ausführungsform der Membran 12 zu entnehmen. Die im Schnitt dargestellte Membran 12 ist zweilagig aufgebaut. Eine erste Lage 42 besteht aus einer etwa 0,02 bis 0,1 mm dicken Folie aus Polyethylen. Die erste Lage 42 wird mit einer zweiten Lage 44 beschichtet, die die haftungsvermittelnde Oberflächenstruktur 20 aufweist. Die zweite Lage 44 besteht aus Silikon und ist 0,02 bis 0,2 mm dick. Hier weist die haftungsvermittelnde Oberflächenstruktur 20 eine Vielzahl von stäbchenförmigen Fortsätzen auf.

## Patentansprüche

1. Implantierbarer Sensor (10) zur intrakorporalen Fixierung an Gewebe (30) mittels Trockenadhäsivität, umfassend:
ein Gehäuseteil (16),
ein plastisch verformbares Gel,
eine Membran (12) und
ein Fixiermittel (12, 20),
wobei
der das Fixiermittel tragende Gehäuseteil (16) des Sensors (10) eine Kavität (18) aufweist, die von der Membran (12) hermetisch abgedeckt wird und wobei das Innere der Kavität (18) mit dem plastisch verformbaren Gel gefüllt ist, so dass sich die Membran (12) beim Andrücken des Sensors (10) an die Oberflächenkontur des Gewebes (30) anpasst und wobei sich an der Außenseite der Membran (12) eine haftvermittelnde Oberflächenstruktur (20) zur Anbindung an das Gewebe (30) befindet.

2. Implantierbarer Sensor (10) nach einem der vorhergehenden Ansprüche, bei dem die haftvermittelnde Oberflächenstruktur (20) aus einem polymeren Werkstoff geformt ist.

3. Implantierbarer Sensor (10) nach Anspruch 2, bei dem der polymere Werkstoff ein Silikon ist.

4. Implantierbarer Sensor (10) nach einem der vorhergehenden Ansprüche, bei dem die Membran (12) mehrlagig aufgebaut ist und die äußerste Lage die haftvermittelnde Oberflächenstruktur (20) aufweist.

5. Implantierbarer Sensor (10) nach Anspruch 4, bei dem die eine oder mehreren inneren Lagen der Membran (12) aus einem polymeren Werkstoff geformt sind.

6. Implantierbarer Sensor (10) nach Anspruch 5, bei dem der polymere Werkstoff ein Polyethylen ist.

7. Implantierbarer Sensor (10) nach einem der vorhergehenden Ansprüche, bei dem die haftvermittelnde Oberflächenstruktur (20) eine Geckostruktur ist.

## Claims

1. An implantable sensor (10) for intracorporeal fixing to tissue (30) by means of dry adhesivity, comprising:
a housing part (16),
a plastically deformable gel,
a membrane (12), and
a fixing means (12, 20),
wherein the housing part (16) of the sensor (10) carrying the fixing means has a cavity (18), which is hermetically covered by the membrane (12), and wherein the interior of the cavity (18) is filled with the plastically deformable gel, such that the membrane (12) adapts to the surface contour of the tissue (30) when the sensor (10) is pressed against the tissue, and wherein an adhesion-promoting surface structure (20) is located on the outer side of the membrane (12) for attachment to the tissue (30).

2. The implantable sensor (10) according to the preceding claim, in which the adhesion-promoting surface structure (20) is formed from a polymer material.

3. The implantable sensor (10) according to claim 2, in which the polymer material is a silicone.

4. The implantable sensor (10) according to any one of the preceding claims, in which the membrane (12) is constructed in a number of layers and the outermost layer comprises the adhesion-promoting surface structure (20).

5. The implantable sensor (10) according to claim 4, in which the one or more inner layers of the membrane (12) is/are formed from a polymer material.

6. The implantable sensor (10) according to claim 5, in which the polymer material is a polyethylene.

7. The implantable sensor (10) according to any one of the preceding claims, in which the adhesion-promoting surface structure (20) is a gecko structure.

## Revendications

1. Capteur implantable (10) destiné à une fixation intracorporelle sur du tissu (30) au moyen d'une adhésivité à sec, comprenant :
une partie boîtier (16),
un gel déformable de manière plastique,
une membrane (12), et
un moyen de fixation (12, 20),
où
la partie boîtier (16) du capteur (10) portant le moyen de fixation présente une cavité (18), qui est recouverte hermétiquement par la membrane (12) et où l'intérieur de la cavité (18) est rempli avec le gel déformable de manière plastique, de sorte que la membrane (12) s'adapte au contour de la surface du tissu (30) lors du plaquage du capteur (10) et où une structure de surface (20) favorisant l'adhésion se trouve sur la face extérieure de la membrane (12) pour une liaison sur le tissu (30).

2. Capteur implantable (10) selon l'une des revendications précédentes, chez lequel la structure de surface (20) favorisant l'adhésion est formée à base d'un matériau polymère.

3. Capteur implantable (10) selon la revendication 2, chez lequel le matériau polymère est un silicone.

4. Capteur implantable (10) selon l'une des revendications précédentes, chez lequel la membrane (12) est construite en couches multiples et la couche la plus extérieure présente la structure de surface (20) favorisant l'adhésion.

5. Capteur implantable (10) selon la revendication 4, chez lequel la ou les couches intérieures de la membrane (12) sont formées à base d'un matériau polymère.

6. Capteur implantable (10) selon la revendication 5, chez lequel le matériau polymère est un polyéthylène.

7. Capteur implantable (10) selon l'une des revendications précédentes, chez lequel la structure de surface (20) favorisant l'adhésion est une structure de gecko.
